# EUROPEAN PATENT APPLICATION

(11) **EP 1 284 121 A2**
(43) Date of publication of application: **19.02.2003**
(21) Application number: 02255327.5
(22) Date of filing: 30.07.2002
(51) Int. Cl.: A61B 5/15

(54) **Physiological sample collection devices and methods of using the same**

(30) Priority: 06.08.2001 US 923093
(71) Applicant: Lifescan, Inc., Milpitas, CA 95035 (US)
(72) Inventor: Yuzhakov, Vadim V., San Jose, CA 95127 (US); McAllister, Devin V., San Jose, CA 95112 (US)
(74) Representative: Mercer, Christopher Paul

(57) **Abstract**

Devices and methods are provided for piercing the skin and accessing and collecting physiological sample therein. The subject devices include at least one fluid pathway, wherein at least a substantial portion of the distal end of the at least one fluid pathway is open to the outside environment. One or more subject devices may be integrated into a test strip for determining the concentration of at least one analyte in the sample. Also provided are methods for using the subject devices. The devices and methods are particularly suited for collecting physiological sample and determining glucose concentrations therein and, more particularly, glucose concentrations in blood, blood fractions or interstitial fluid. Also provided are kits that include the devices for use in practicing the subject methods.

## Description

### FIELD OF THE INVENTION

The field of this invention is analyte concentration determination, particularly physiological sample concentration determination and more particularly glucose concentration determination.

### BACKGROUND OF THE INVENTION

Analyte concentration determination in physiological samples is of ever increasing importance to today's society. Such assays find use in a variety of application settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like. In response to this growing importance of analyte concentration determination, a variety of analyte concentration determination protocols and test strips for both clinical and home testing have been developed.

However, to determine the concentration of an analyte in a physiological sample, a physiological sample must first be obtained. Obtaining the sample oftentimes involves cumbersome and complicated devices which may not be easy to use or may be costly to manufacture. Furthermore, the procedure for obtaining the sample may also be painful, where the pain may be compounded where the skin need be pierced multiple times to find a suitable sample site to obtain the requisite sample volume. For example, pain is often associated with the size of the needle used to obtain the physiological sample and the depth to which the needle is inserted. Depending on the analyte and test employed, a relatively large, single needle or the like is often used to extract the requisite amount of sample. Furthermore, these single needles only collect sample located at the distal tip of the needle, i.e., at the opening located at the distal tip, thus preventing sampling from adjacent sites such as adjacent capillary beds, without multiple needle penetrations. Still further, the process may involve a multitude of steps which increase the test time. For example, a patient may be required to activate a skin-piercing mechanism to pierce the skin and then activate a sample collection mechanism to collect the sample from the punctured site. The sample must then be transferred to a testing device, e.g., a test strip or the like, and then oftentimes the test strip is then transferred to a measuring device such as a meter. A patient then must wait for the measuring device to generate and display an analyte concentration reading. Because of these disadvantages, it is not uncommon for patients who require frequent monitoring of an analyte to simply avoid monitoring the analyte of interest. With diabetics, for example, the failure to measure their glucose level on a prescribed basis results in a lack of information necessary to properly control the level of glucose. Uncontrolled glucose levels can be very dangerous and even life threatening.

In order to simplify the analyte sampling and measuring processes, attempts have been made to combine a lancing-type device with various other components involved in analyte concentration determination. For example, U.S. Patent No. 6,099,484 discloses a sampling device which includes a single needle associated with a spring mechanism, a capillary tube associated with a pusher and a test strip. An analyzer may also be mounted in the device for analyzing the sample. Accordingly, the single needle is displaced toward the skin surface by un-cocking a spring and then retracting it by another spring. A pusher is then displaced to push the capillary tube in communication with a sample and the pusher is then released and the fluid is transferred to a test strip.

U.S. Patent No. 5,820,570 discloses an apparatus which includes a base having a hollow needle and a cover having a membrane, whereby the base and cover are connected together at a hinge point. When in a closed position, the needle is in communication with the membrane and fluid can be drawn up through the needle and placed on the membrane of the cover.

While effective, there are drawbacks associated with each of the above devices and techniques. For example, the devices disclosed in the aforementioned patents utilize complex components, thus decreasing ease-of-use and increasing manufacturing costs. Furthermore, as described, a single needle design may be associated with increased pain because the conventional configured single needle must be relatively large to extract the requisite sample size. Also, the needle only collects sample from sites at its distal tip. Still further, in regards to the system of the '484 patent, the steps of activating and retracting a needle and then activating and retracting a capillary tube adds still more user interaction, increases test times and decreases ease-of-use.

As such, there is continued interest in the development of new devices and methods for use in the determination of analyte concentrations in a physiological sample. Of particular interest would be the development of devices, and methods of use thereof, that are efficient, involve minimal pain, are simple to use, have short overall test times, can access alternative sampling sites and which may be used with various analyte concentration determination systems.

### SUMMARY OF THE INVENTION

Devices and methods are provided for piercing the skin and accessing and collecting physiological sample therein. The subject devices include at least one fluid pathway, wherein at least a substantial portion of the distal end of the at least one fluid pathway is open to the outside environment. One or more subject devices may be integrated into a test strip for determining the concentration of at least one analyte in the sample. Also provided are methods for using the subject devices. The devices and methods are particularly suited for collecting physiological sample and determining glucose concentrations therein and, more particularly, glucose concentrations in blood, blood fractions or interstitial fluid. Also provided are kits that include the devices for use in practicing the subject methods.

### BRIEF DESCRIPTIONS OF THE DRAWINGS

Figure 1A shows an exemplary embodiment of a skin-piercing element according to the subject invention.
Figure 1B shows another exemplary embodiment of the subject skin-piercing elements.
Figure 1C shows an exemplary embodiment of the subject invention having a plurality of skin-piercing elements of Figure 1A.
Figure 1D shows an exemplary embodiment of a subject skin-piercing element having two fluid pathways.
Figure 1E shows an exemplary embodiment of a subject skin-piercing element having a fluid pathway that diverges into two separate paths.
Figure IF shows an exemplary embodiment of a subject skin-piercing element having a plurality of fluid pathways.
Figure 2A shows another exemplary embodiment of a subject skin-piercing element having a portion of the fluid pathways closed to the outside environment, wherein the fluid pathway is associated with openings to collect fluid.
Figure 2B shows an exemplary embodiment of the subject skin-piercing element having a plurality of openings associated with a fluid pathway.
Figure 2C shows an exemplary embodiment of the subject skin-piercing element having a plurality of openings associated with a plurality of fluid pathways.
Figure 3A shows an exemplary embodiment of a test strip having a plurality of subject skin-piercing elements associated with it.
Figure 3B shows an exemplary embodiment of a test strip having a plurality of subject skin-piercing elements associated with it.
Figure 4 shows an embodiment of a meter of the present invention for use with the test strips of the present invention.

### DETAILED DESCRIPTION OF THE INVENTION

Devices and methods are provided for piercing the skin and accessing and collecting physiological sample therein. The subject devices include at least one fluid pathway, wherein at least a substantial portion of the distal end of the at least one fluid pathway is open to the outside environment. One or more subject devices may be integrated into a test strip for determining the concentration of at least one analyte in the sample. Also provided are methods for using the subject devices. The devices and methods are particularly suited for collecting physiological sample and determining glucose concentrations therein and, more particularly, glucose concentrations in blood, blood fractions or interstitial fluid. Also provided are kits that include the devices for use in practicing the subject methods. In further describing the subject invention, the subject devices will be described first, followed by a review of the subject methods for use in practicing the subject devices.

Before the present invention is described, it is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. Thus, for example, reference to "a test strip" includes a plurality of such test strips and reference to "the reagent" includes reference to one or more reagents and equivalents thereof known to those skilled in the art, and so forth.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEVICES

As summarized above, the subject invention provides devices for piercing the skin and accessing and collecting a physiological sample therein. More specifically, the subject invention provides skin-piercing elements, where the skin piercing elements have at least one fluid pathway, wherein at least a substantial portion of the distal end of the at least one fluid pathway is open to the outside environment. In certain embodiments, at least a substantial portion of the distal end of the skin-piercing element is open to the outside environment, where a substantial portion may indicate a substantial portion of one or more side or along any point of the circumference of the at least one fluid pathway. In many embodiments of the subject skin-piercing elements, the at least one fluid pathway terminates proximal to the distal tip of the skin-piercing element to which it is associated.

One or more subject skin-piercing elements may be associated or integrated with a test strip for determining at least one target analyte concentration in the sample. The subject test strips find use in the determination of a wide variety of different analyte concentrations, where representative analytes include, but are not limited to, glucose, cholesterol, lactate, alcohol, and the like. In many embodiments, the subject test strips are used to determine the glucose concentration in a physiological sample, e.g., interstitial fluid, blood, blood fractions, constituents thereof, and the like. While it is to be understood that a variety of different types of test strips may be suitable for use with the present invention, e.g., colorimetric and electrochemical test strips, the subject invention will be described herein in reference to an electrochemical test strip, where such description is by way of example and not limitation.

### Skin Piercing Element

As described above, a feature of the subject invention is a skin-piercing element which advantageously allows for the access and collection of a physiological sample with minimal pain. In other words, pain is minimized due to the particular shape and configuration of the skin-piercing element which allows the distal tip to be smaller and/or sharper (e.g., smaller cross sections and sharper tips) and enables access to greater area for sample collection, compared to currently configured skin-piercing elements.

Accordingly, the skin-piercing elements of the subject invention are configured for piercing the skin. More particularly, the skin-piercing elements are configured to pierce the skin and draw or collect physiological sample therefrom. To this end, each skin-piercing element has a distal end having a sharp distal tip. Furthermore, each of the skin-piercing elements has at least one fluid pathway through which the fluid sample travels, typically due to capillary forces. The fluid pathway may terminate proximal to the distal tip of the skin-piercing element to which it is associated, where such a configuration imparts structure to the distal tip while enabling the tip to be sufficiently sharp.

Any suitable shape of the skin-piercing elements may be employed, as long as the shape enables the skin to be pierced, and more particularly pierced with minimal pain to the patient or user of the test strip. For example, the skin-piercing elements may be substantially cylindrical-like, wedge-like, triangular in shape such as a substantially flattened triangle-like configuration, blade shaped, or any other suitable shape. The cross-sectional shape of the skin-piercing element, or at least the portion of skin-piercing element that is penetrable into the skin, may be any suitable shape, including, but not limited to, substantially rectangular, square, oval, circular, diamond, triangular, star, etc. To provide minimal pain to the user, the tips of the skin-piercing elements, i.e., the distal tips, are also suitably shaped to pierce the skin. For example, the distal tips are sufficiently small and/or sharp to enable piercing and penetration of the skin with minimal pain. As such, the skin-piercing elements may be tapered or may otherwise define a point or apex at the end of the skin-piercing elements. Such a configuration may take the form of an oblique angle at the tip or a pyramid or triangular shape or the like.

The dimensions of the skin-piercing elements may vary depending of a variety of factors such as the type of physiological sample to be obtained and the desired penetration depth and the thickness of the skin layers of the particular patient being tested. Generally, the skin-piercing elements are constructed to provide skin-piercing and fluid extraction functions and thus will be designed to be sufficiently robust to withstand insertion into and withdrawal from the skin. Typically, to accomplish these goals, the ratio of the penetration length (defined by the distance between the base of the skin-piercing element and its distal tip) to diameter (where such diameter is measured at the base of the skin-piercing element) ranges from about 1 to 1, usually about 2 to 1, more usually about 5 to 1 or 10 to 1 and oftentimes 50 to 1. The height or thickness of the skin-piercing element, at least the thickness of the distal portion of the skin-piercing element, typically ranges from about 1 to 1000 microns, usually from about 10 to 500 microns and more usually from about 50 to 250 microns.

The total length of the skin-piercing element typically ranges from about 1 to 30,000 microns, usually from about 100 to 10,000 microns and more usually from about 1,000 to 3,000 microns. The penetration length of the skin-piercing elements, i.e., the length that is penetrable into the skin (the distal portion of the skin-piercing element) generally ranges from about 1 to 5000 microns, usually about 100 to 3000 microns and more usually about 1000 to 2000 microns. The proximal portion of the skin-piercing element typically ahs a length that ranges from about 1 to 5000 microns, usually about 100 to 3000 microns and more usually about 1000 to 2000 microns. In many embodiments, the outer diameter of the distal tip generally does not exceed about 100 microns and is generally less than about 20 microns and more typically less than about 1 micron. The outer diameter at the base generally ranges from about 1 to 2000 microns, usually about 300 to 1000 microns and more usually from about 500 to 1000 microns. However, it will be appreciated by one of skill in the art that the outer diameter of the skin-piercing element may vary along its length or may be substantially constant.

The skin-piercing elements will typically be manufactured of a biocompatible material, usually material which can impart the desired rigidity for piercing and penetrating skin and obtaining sample without breaking or substantially flexing. Materials suitable for use in the subject invention include, but are not limited to, metals and alloys such as stainless steel, palladium, titanium, and aluminum, plastics such as polyetherimide, polycarbonate, polyetheretherketone, polyimide, polymethylpentene, polyvinylidene fluoride, polyphenylsulfone, liquid crystalline polymer, polyethylene terephthalate (PET), polyethylene terephthalate, glycol modified (PETG), polyimide and polycarbonate and ceramics such as silicon and glass. In many embodiments, the above mentioned materials may further include particles, e.g., micro or nano particles or fibers, of a suitable metal, carbon siliceous material, e.g., glass, or ceramic. A suitable insulating material such as polyethylene terephthalate (PET), polyethylene terephthalate, glycol modified (PETG), polyimide, polycarbonate, polystyrene, silicon, silicon dioxide, ceramic, glass, and the like may also be included. Of particular interest is the use of chemical vapor deposited SiO₂ as an insulating layer due to its hydrophilic nature which may facilitate fluid sample collection.

As described above, a feature of the skin-piercing element of the subject invention is the presence of at least one fluid pathway therein for collecting and transferring physiological sample accessed by the skin-piercing element. In certain subject devices, the at least one fluid pathway terminates proximal to the distal tip of the skin-piercing element, i.e., the fluid pathway does not extend all the way to the distal tip or apex of the skin-piercing element. The at least one fluid pathway has at least a substantial portion of its distal end open to the outside environment such that either at least one of its sides is open to the outside environment or it is open to the outside environment along any point of the circumference of the at least one fluid pathway or one or more openings or holes positioned in the distal portion of the skin-piercing element are open to the outside environment. By substantial portion is means about 1 to 99% of the total surface area of the distal end of the fluid pathway, usually about 50 to 95% and more usually about 80 to 90%. Of course, the entire length of at least one side, oftentimes as much as the entire length of at least two sides, of the distal portion of the fluid pathway may be open to the outside environment, where the entire length of at least two sides of the total length of the fluid pathway may be open to the outside. In certain embodiments of the subject invention, the skin-piercing element includes a plurality of fluid pathways, where the ratio of holes to fluid pathways may correspond to about a 1 to 1 ratio, about a 1 to 2 ratio, or greater such as about 1 to 1000 ratio. Similarly, the ratio of openings to pathways may be about 2 to 1, about 3 to 1, or any suitable combination such as about 1000 to 1.

The fluid pathway of the subject invention may be dimensioned to provide a capillary force or effect upon the physiological sample, such that the capillary effect draws or wicks physiological sample into the skin-piercing element, and oftentimes then into an associated test strip as will be described in greater detail below. As will be apparent to one of skill in the art, the dimensions of the fluid pathway will vary depending on a number of factors, including the presence of a single fluid pathway or a plurality of channels. However, typically, the diameter or width of a single fluid pathway generally will not exceed 1000 microns and will usually be about 100 to 200 microns in diameter. The diameter of the fluid pathway may be constant along its length or may vary. Similarly, the total length of a single fluid pathway will vary depending on a variety of factors, but will typically be about 1 to 99% of the total length of the corresponding skin-piercing element, usually about 50 to 99% and more usually about 70 to 99% of the total length of the skin-piercing element. As such, the distal portion length may vary, but typically is about 1 to 99% of the of the total length of the corresponding skin-piercing element, usually about 1 to 50% and more usually about 1 to 30% of the total length of the skin-piercing element.

In certain embodiments, the fluid pathway may further include one or more agents to facilitate sample collection. For example, one or more hydrophilic agents may be present in the fluid pathway, where such agents include, but are not limited to types of surfactants such as MESA, Triton, Macol, Tetronic, Silwet, Zonyl and Pluronic.

The skin-piercing elements of the present invention may be fabricated using any convenient technique including, but not limited to, known techniques in the art such as microreplication techniques including embossing, injection molding and casting processes, where embossing techniques are of particular interest. Such techniques, and in particular embossing techniques, enable low cost manufacture and also advantageously enable the distal tip of the skin-piercing element to be near infinitesimally small, e.g., the cross-sectional area is small, and sharp. Furthermore, embossing techniques allow precise, consistent fabrication of the subject skin-piercing elements.

In such an embossing technique, a precursor material such as a suitable thermoplastic precursor material, as described above, having a thickness in the range of about 25 to 650 microns, usually from about 50 to 625 microns and more usually from about 75 to 600 microns is placed into an embossing apparatus, where such an apparatus includes a mold having features, often times a negative image of the features, of the skin-piercing element. The precursor material is then compressed by the mold under heat and a suitable compression force. Usually, a temperature in the range from about 20°C to 1500°C is used, usually from about 100°C to 1000°C and more usually from about 200°C to 500°C. Heat is usually applied for about 0.1 to 1000 seconds, usually about 0.1 to 100 seconds and more usually about 0.1 to 10 seconds. The compression force is usually applied in the range from about 1 to 50 GPA is used, usually from about 10 to 40 GPA and more usually from about 20 to 30 GPA. The compression force is usually applied for about 0.1 to 100 seconds, usually about 0.1 to 10 seconds and more usually about 0.1 to 1 seconds. The heat and compression force may be applied at the same or different times. After the material is cooled, it is removed from the apparatus, and post processing may then occur, if necessary. For example, surface modifications such as hydrophobicity or hydrophilicity may be added, openings or holes may be drilled (often times by laser, hot cutting technique as are known in the art, or the like), metalization of certain areas to create electrodes, etc. It will be apparent that the above-described method of manufacture may be used to fabricate a plurality of skin-piercing elements from a single precursor material, such that following the cooling, the material may then be cut into a plurality of skin-piercing elements. Of course, the methods may also be used to manufacture a single skin-piercing element.

Embodiments of the subject invention will now be described in greater detail in reference to the drawings where like numerals refer to like features or components.

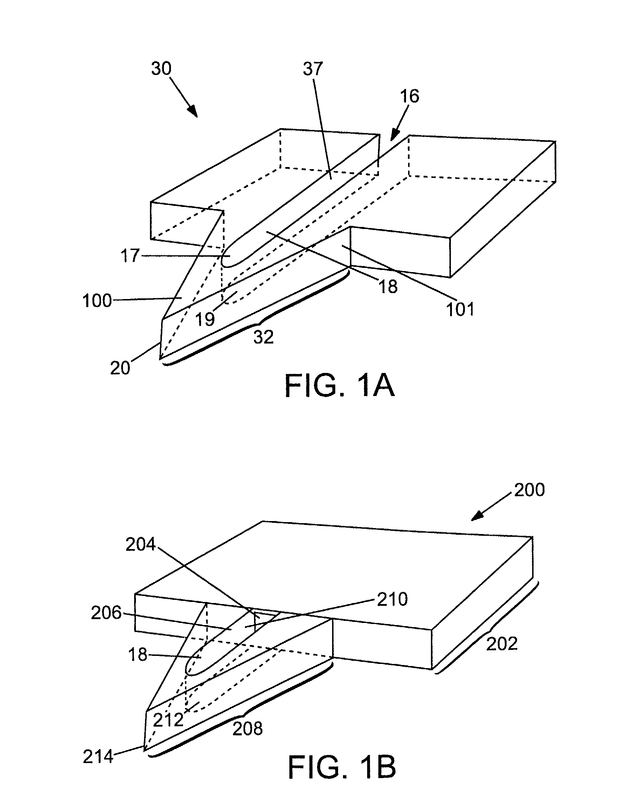

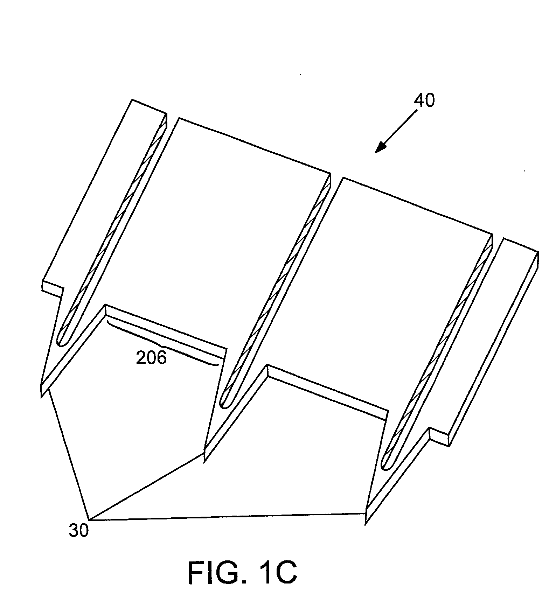

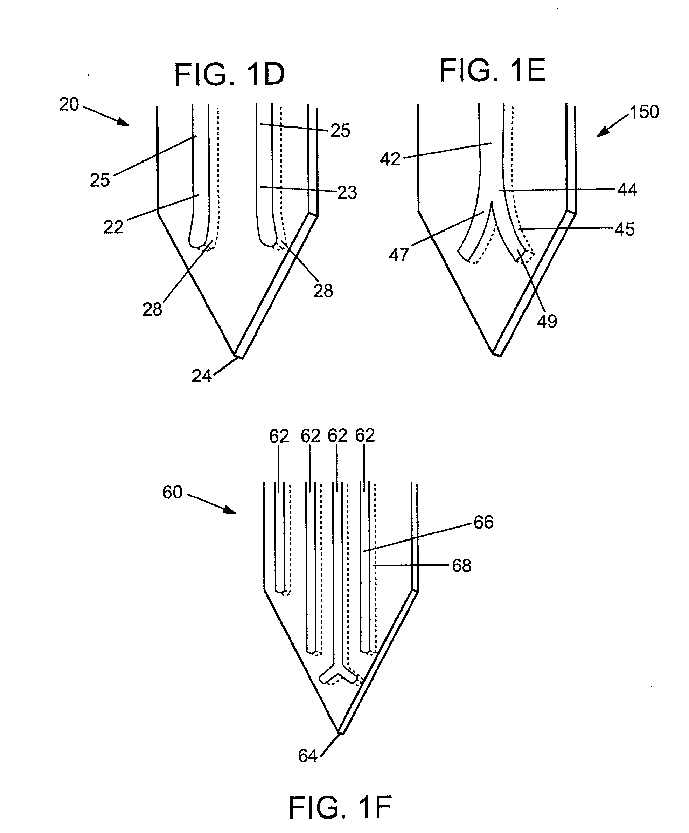

Referring to the figures, Figure 1 shows an exemplary embodiment of the subject skin-piercing element having a fluid pathway therein, where at least a substantial portion of the distal end of the fluid pathway is open to the outside environment, e.g., on at least one side or along at least a first portion and/or a second portion of the circumference of the fluid pathway. Oftentimes, the entire length of the fluid pathway is open to the environment, usually on at least two sides or two points or portions along the circumference, e.g., two opposing sides or portions to define a slit or through-groove in the skin-piercing element. In this embodiment, skin-piercing element 30 includes fluid pathway or channel 16 having a distal portion 18 and a proximal portion 37, where the fluid pathway 16 terminates proximal to the distal tip 20. As illustrated by the figure, fluid pathway 16 runs through a portion of distal end (penetration length) 32 in such a way that a substantial portion of both the distal portion 18 and the proximal portion 37 of the fluid pathway are open to the outside environment along the entire length of the skin-piercing element. In this particular embodiment, fluid pathway 16 is open to the outside environment on a first side 17 and a second side 19. In other words, the fluid pathway forms a slit or groove through the skin-piercing element. However it will be apparent that it can be open on just one side or portion of a circumference (any one side or portion) or can be open on any combination of sides or along any point of the circumference of the skin-piercing element as well, including sides 100 and 101, i.e., any combination of side openings are contemplated by this invention. Thus, it will be appreciated that this skin-piercing configuration offers several advantages over current needles or lances that enable it to minimize the pain associated with physiological sample access and collection. For example, the ability of the distal tip to be narrowly dimensioned is due to the termination of the fluid pathway proximal thereto. Another important advantage of the skin-piercing elements of the present invention, due to the relatively greater surface area of one or more sides of the skin-piercing element, is the ability to access a greater sampling area and thus provide a greater collection rate of sampling.

Figure 1B shows an exemplary embodiment of another skin-piercing element according to the subject invention. In this embodiment, the fluid pathway 206 of skin-piercing element 200 is open to the outside environment only on the distal portion 208 of the skin-piercing element, i.e., only the distal portion 208 is open to the outside environment on two of its sides, a first side 210 and a second side 212. However, fluid pathway 206 continues through the proximal portion 202 of the skin-piercing element 200, but is not open to the outside environment on this portion, except for the entry point 204 of the fluid pathway 206 into the proximal portion 202 and the exit point (not shown). As with the fluid pathway 16 of Figure 1A, the fluid pathway 206 of skin-piercing element 1B terminates proximal to the distal tip 214 of the skin-piercing element.

Figure 1C shows a device 40 having a plurality of the skin-piercing elements 30 of Figure 1A. The distance 206 between the skin-piercing elements generally ranges from about 200 to 6000 microns, usually from about 200 to 3000 microns and more usually from about 2000 to 3000 microns.

Figure 1D shows another exemplary embodiment of the subject skin-piercing element having a fluid pathway therein, where at least a substantial portion of the distal end of the fluid pathway is open to the outside environment. Skin-piercing element 20 includes two fluid pathways 22 and 23, where both fluid pathways terminate proximal to the distal tip 24. Furthermore, fluid pathways 22 and 23 are open to the outside environment along at least the entire length of a first side 25 and a second side 28 of the distal end of the fluid pathways 22 and 23 (in this embodiment shown as being substantially opposite to a first side 25, but may be one or more other sides or portions of the circumference (in addition to or in place of a side substantially opposite to a first side) as well). The fluid pathways 22 and 23 terminate proximal to the distal tip 24.

Figure 1E shows another exemplary embodiment of the subject invention. In this embodiment, at least a substantial portion of the distal end of fluid pathway 42 of device 150 is open to the outside environment. In this embodiment, the fluid pathway 42 is open along at least its entire distal length on a first side 44 and a second side 45, where the fluid pathway 42 terminates proximal to distal tip 43. In this embodiment, fluid pathway 42 diverges into two separate pathways 47 and 49.

Figure IF shows another exemplary embodiment of the subject invention having at least a substantial portion of the distal end of each fluid pathway open to the outside environment. In this embodiment, skin-piercing element 60 has a plurality of fluid pathways 62 therein. The number of fluid pathways may vary depending on a number of factors, including the particular area to be sampled and the like. Typically, a skin-piercing element may include from about 1 to 50 fluid pathways, usually about 1 to 25 and more usually from 1 to 15 fluid pathways. In this embodiment, each of the plurality fluid pathways 62 terminate proximal to the distal tip 64 and are open to the outside environment on sides 66 and 68 along at least their entire lengths of the distal end.

As mentioned above, the at least a portion of a fluid pathway may not be open and instead may be associated with at least one opening. In certain embodiments, the openings may comprise a substantial portion of the surface area of the distal end of the skin-piercing element, at least on one side or portion along then circumference of the skin-piecing element. The opening(s) may be positioned in a variety of areas of the skin-piercing element, where the exact positioning may depend on a number of factors such as the characteristics and number of the fluid pathway(s), the particular analyte of interest and the site from which sample is to be collected. Typically, the at least one opening will be positioned proximal to the distal tip of the skin-piercing element, as will the at least one fluid pathway, so as to provide the necessary strength to the tip. As noted above, the number of openings to fluid pathways may vary.

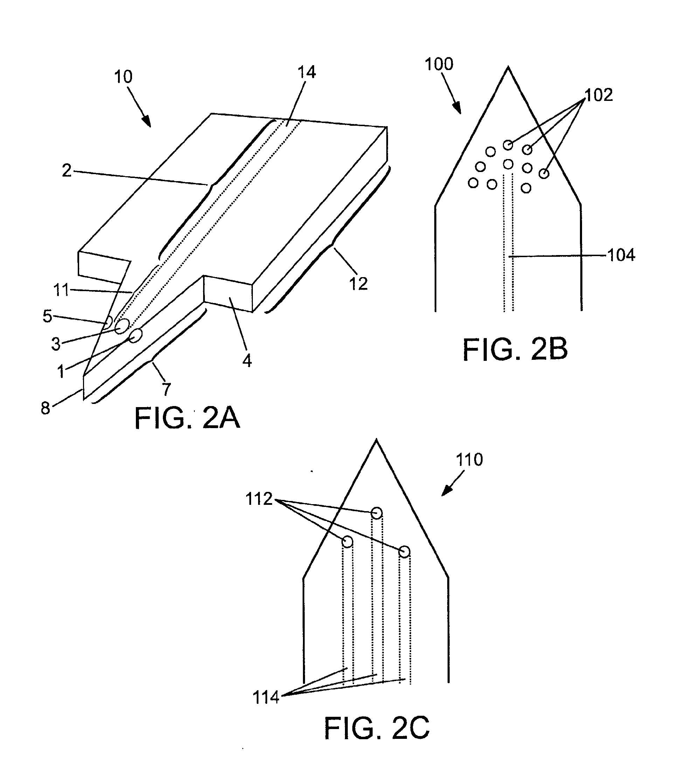

Figure 2A shows an exemplary embodiment of a skin-piercing element having side openings associated with a fluid pathway. In this embodiment, at least a substantial portion of the distal end of the fluid pathway is open to the outside environment via openings associated with the fluid pathway(s). In other words, the fluid pathway(s) terminate at one or more openings in communication with the outside environment. Skin-piercing element 10 has a proximal portion 12 and a distal portion 7 with distal tip 8, where proximal and distal portions are demarcated by juncture or position 4. The fluid pathway 14 is terminated proximal to distal tip 8. The distal portion 11 of the fluid pathway 14 is closed to the environment (the proximal portion 12 of the fluid pathway 14 may be open or closed to the environment, herein shown as closed to the outside environment) except for access to the outside environment via side openings. In other words, the fluid pathway is associated with one or more openings along the skin-piercing element. In the embodiment illustrated in figure 2A, the fluid pathway is associated with openings 1, 3 and 5. However, other openings may exist in addition to or instead of the openings 1, 3 and 5, such as an opening substantially opposite to openings 5. In this embodiment, the fluid pathway 14, as well as any associated holes, are positioned proximal to the distal tip 8.

Figure 2B shows another exemplary embodiment of a skin-piercing element where at least a substantial portion of the distal end of a fluid pathway of a skin-piercing element is open to the outside environment via a plurality of openings. In this embodiment, skin-piercing element 100 has at least one fluid pathway 104 associated with a plurality of openings or holes 102 positioned in the distal portion of the skin-piercing element. Of course, the number of openings may vary, depending on the size of each opening and the like, where the number can be as few as about 1 and as great as about 1000, oftentimes between about 1 to 100 openings. In this particular embodiment, one fluid pathway is shown. However, any number of fluid pathways may be present, such that the ratio of holes to fluid pathways may vary, as described above.

Figure 2C shows another exemplary embodiment of a skin-piercing element according to the subject invention. In this particular embodiment, skin-piercing element 110 has a plurality of openings 112 associated with a plurality of fluid pathways 114. Similar to skin-piercing element 100 of Figure 2B, the sides of the distal portion of the fluid pathways are not open to the outside environment, except for their association with openings 112.

### Test Strip

As described above, one or more skin-piercing elements of the subject invention may be associated with a test strip for determining the concentration of at least one analyte in a physiological sample. While it is to be understood that a variety of test strips may be used with the subject invention, e.g., electrochemical and colorimetric or photometric (colorimetric and photometric are herein used interchangeably), the subject invention will be described herein in reference to an electrochemical test strip, where such description will be by way of example and not limitation.

Generally, the test strip, e.g., an electrochemical test strip, is made up of two opposing metal electrodes separated by a thin spacer layer, where the test strip includes at least one reaction area or zone and where at least one subject skin-piercing element, and often a plurality of skin-piercing elements, is associated or integrated with the test strip, as will be further described below. In many embodiments a redox reagent system is located in the reaction area or zone. The test strips may be configured and adapted to be received into a meter for automatically determining the concentration of at least one analyte in a physiological sample, or may be of any convenient shape and configuration.

In certain embodiments of these electrochemical test strips, the working and reference electrodes are generally configured in the form of elongated rectangular strips, but may be any appropriate shape or configuration. Typically, the length of the electrodes ranges from about 1.9 to 4.5 cm, usually from about 2.0 to 2.8 cm. The width of the electrodes ranges from about 0.07 to 0.8 cm, usually from about 0.20 to 0.60 cm. The working and reference electrodes typically have a thickness ranging from about 10 to 100 nm and usually from about 10 to 20 nm.

The working and reference electrodes are further characterized in that at least the surfaces of the electrodes that face the reaction area of the electrochemical cell in the strip is a metal, where metals of interest include palladium, gold, platinum, silver, iridium, carbon (conductive carbon ink), doped tin oxide, stainless steel and the like. In many embodiments, the metal is gold or palladium.

While in principle the entire electrode may be made of the metal, each of the electrodes is generally made up of an inert support material on the surface of which is present a thin layer of the metal component of the electrode. In these more common embodiments, the thickness of the inert backing material typically ranges from about 25 to 500, usually 50 to 400 µm, while the thickness of the metal layer typically ranges from about 10 to 100 nm and usually from about 10 to 40 nm, e.g. a sputtered metal layer. Any convenient inert backing material may be employed in the subject electrodes, where typically the material is a rigid material that is capable of providing structural support to the electrode and, in turn, the electrochemical test strip as a whole. Suitable materials that may be employed as the backing substrate include plastics, e.g., polyethylene terephthalate (PET), polyethylene terephthalate, glycol modified (PETG), polyimide, polycarbonate, polystyrene, silicon, ceramic, glass, and the like.

A feature of the subject electrochemical test strips is that the working and reference electrodes as described above generally face each other and are separated by only a short distance, such that the spacing between the working and reference electrodes in the reaction zone or area of the electrochemical test strip is extremely narrow. This minimal spacing of the working and reference electrodes in the subject test strips is a result of the presence of a thin spacer layer positioned or sandwiched between the working and reference electrodes. The thickness of this spacer layer may range from 50 to 750 µm and is often less than or equal to 500 µm, and usually ranges from about 100 to 175 µm.

In certain embodiments, the spacer layer is configured or cut so as to provide a reaction zone or area, where in many embodiments the volume of the reaction area or zone formed by the spacer layer typically ranges from about 0.1 to 10 µL, usually from about 0.2 to 5.0 µL. However, as described below, the reaction area may include other areas or be elsewhere all together, such as in a fluid pathway or the like. The spacer layer may have a circular reaction area, or other configurations, e.g., square, triangular, rectangular, irregular shaped reaction areas, etc., and may be cut with side inlet and outlet vents or ports The spacer layer may be fabricated from any convenient material, where representative suitable materials include polyethylene terephthalate (PET), polyethylene terephthalate, glycol modified (PETG), polyimide, polycarbonate, and the like, where the surfaces of the spacer layer may be treated so as to be adhesive with respect to their respective electrodes and thereby maintain the structure of the electrochemical test strip.

Regardless of where the reaction zone is, in many embodiments, a reagent system or composition is present in the reaction area, where the reagent system interacts with components in the fluid sample during the assay.

Reagent systems of interest typically include a redox couple. The redox couple of the reagent composition, when present, is made up of one or more redox couple agents. A variety of different redox couple agents are known in the art and include: ferricyanide, phenazine ethosulphate, phenazine methosulfate, pheylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, ruthenium complexes, and the like. In many embodiments, redox couples of particular interest are ferricyanide, and the like.

Other reagents that may be present in the reaction area include buffering agents, e.g. citraconate, citrate, malic, maleic, phosphate, "Good" buffers and the like. Yet other agents that may be present include: divalent cations such as calcium chloride, and magnesium chloride; surfactants such as Triton, Macol, Tetronic, Silwet, Zonyl, and Pluronic; stabilizing agents such as albumin, sucrose, trehalose, mannitol, and lactose.

Examples of such a reagent test strips suitable for use with the subject invention include those described in EP-A-1 067 384, WO 01/57510, WO 01/57238,

Generally for electrochemical assays, an electrochemical measurement is made using the reference and working electrodes. The electrochemical measurement that is made may vary depending on the particular nature of the assay and the test strip with which the electrochemical test strip is employed, e.g., depending on whether the assay is coulometric, amperometric or potentiometric. Generally, the electrochemical measurement will measure charge (coulometric), current (amperometric) or potential (potentiometric), usually over a given period of time following sample introduction into the reaction area. Methods for making the above described electrochemical measurement are further described in U.S. Patent Nos.: 4,224,125; 4,545,382; and 5,266,179; as well as WO 97/18465; WO 99/49307; the disclosures of which are herein incorporated by reference. Regardless of the type of measurement, an electrochemical measurement or signal is made in the reaction zone of the test strip.

Following detection of the electrochemical measurement or signal generated in the reaction zone as described above, the amount of the analyte present in the sample introduced into the reaction zone is then determined by relating the electrochemical signal to the amount of analyte in the sample. As described above, the test strips are configured and adapted to be received by a meter. Representative meters for automatically practicing these steps are further described in EP-A-1 067 384, WO 01/57510, WO 01/57238.

Of course, in those embodiments using a colorimetric assay system, a spectrophotometer or optical meter will be employed, where representative meters are further described in, for example, U.S. Patent Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference.

As noted above, at least one subject skin-piercing element is associated with the test strip, i.e., is integral with or a part of the test strip. The at least one skin-piercing element may be manufactured as a separate component or piece which is then affixed or attached to the test strip or it may be manufactured as a part of the test strip, as will be described in more detail below.

In those embodiments where the skin-piercing elements are manufactured as a separate component or piece, they are associated or attached to the test strip by an convenient means. For example, any suitable adhesive may be used, as is commonly known in the art.

In certain embodiments, e.g., where the plurality of skin-piercing elements is positioned substantially parallel to the test strip, the plurality of skin-piercing elements may be made of the same material as the test strip, i.e., a unitary construction or a single piece of material. In other words, the plurality of skin-piercing elements may be formed of or from the spacer layer of the test strip, for example, such that the plurality of skin-piercing elements and the test strip are one piece of material.

The test strip itself may form a portion of the fluid pathway of the subject skin-piercing element. In other words, the test strip may provide one or more barriers or walls of the fluid pathway to confine sample that is traveling through the fluid pathway between the test strip and the fluid pathway walls. For example, a portion of the proximal portion of the fluid pathway may be made of or confined by the test strip. More specifically, where the fluid pathway is open to the outside environment along at least a portion of its length, for example a proximal portion of its length, a part of the test strip may then form a cover or wall over the open portion of the fluid pathway. In certain embodiments of the subject devices, one or both electrodes form the barrier(s). For example, a portion of the fluid pathway may be formed by the material of skin-piercing element, e.g., the material of the spacer layer, and one or both electrodes may then provide additional barriers to the fluid pathway such that sample is in contact with one or more electrodes of the test strip as it is being wicked or passed through the fluid pathway. In many embodiments, one or more testing reagents, such as reagents of a redox reagent system, will be present or positioned in the fluid pathway, in addition to, or instead of, in other locations or other reaction areas of the test strip. Accordingly, it will be appreciated by one of skill in the art that the reaction, i.e., analyte concentration determination, will occur or commence sooner than if the sample had to travel to a remote reaction zone before the reaction could commence. Thus, the accuracy of analyte measurement is increased and the reaction time, i.e., the time it takes to generate the concentration of analyte in the sample, is decreased.

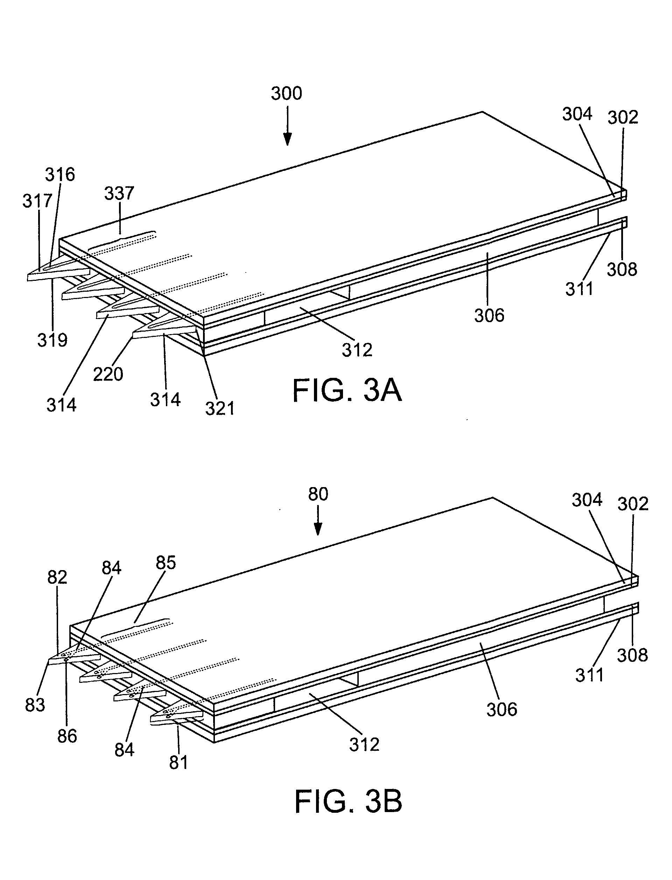

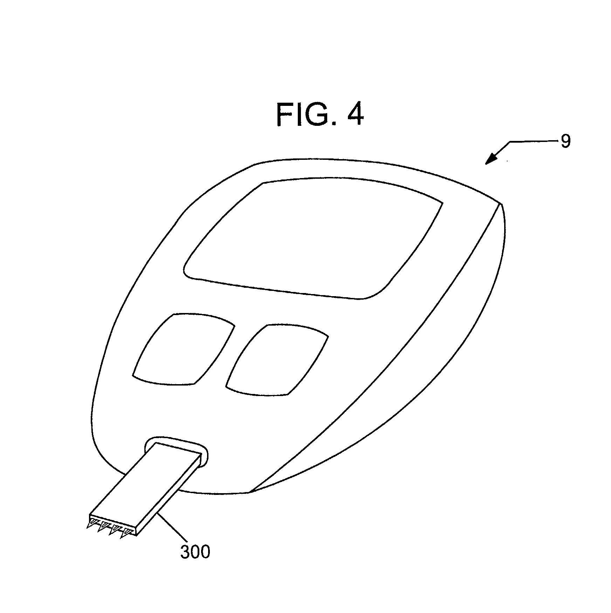

Referring again to the figures, where like numerals refer to like components, Fig. 3A shows a representative test strip 300 according to the subject invention having a plurality of skin-piercing elements associated therewith. Test strip 300 includes a first electrode 302 with an associated inert backing 304 and a second electrode 308 with an associated inert backing 311. As described above, the test strip 300 has a spacer layer 306, where in this embodiment spacer layer306, along with electrodes 302 and 308, define a reaction area 312. Test strip 300 is configured and adapted to be inserted into a meter. More specifically, the test strip has a first end and a second end, wherein the plurality of skin-piercing elements is associated with at least the first end and at least the second end is configured for insertion into a meter 9, as seen in Figure 4.

Test strip 300 also includes a plurality of skin-piercing elements 314, where such skin-piercing element may be made of the same material as the spacer layer 306 or of a different material. It will be apparent that test strip 300 may include any number of skin-piercing elements, where such numbers may range from about 1 to 50, usually from about 1 to 25. Skin-piercing elements 314 have fluid channels 316 which are open along the entire lengths of a first side 317 and a second side 319 to the outside environment. The fluid pathways 314 also terminate proximal to distal tips 320. The penetration length or distal portion of the skin-piercing elements 314 is shown as the distance between base 321 and distal tip 320.

The proximal portion 337 of fluid pathways 316 is positioned between the two electrodes 302 and 308 and is herein illustrated by dashed lines to indicate that the test strip confines the fluid pathways therebetween. Accordingly, the electrodes of the test strip form barriers or walls for a portion of the fluid pathways such that sample flowing through the pathways is contacted by the electrodes while still in the pathway. As described above, an appropriate redox reagent system may be located in the fluid pathways to define another reaction area or zone of the test strip. In certain other embodiments, proximal portion 337 of the fluid pathways may not be open, i.e., in contact with the electrodes along their length.

Figure 3B shows another exemplary embodiment of the subject invention. In this embodiment, test strip 80 includes a plurality of skin-piercing elements 82, each having fluid pathways or channels 84 therein which terminate and are in fluid communication with associated openings 86 at the distal end 81 of the skin-piercing element 80. Each fluid pathway 84 terminates proximal to the distal tip 83 of the skin-piercing element 82 to which it is associated. Fluid pathways 84 are embedded or run through the distal portion such that at least this portion of the fluid pathways is closed to the outside environment, except for the openings 86. In the particular embodiment, the fluid pathways 84 are associated with two openings on opposing sides of the distal end. The proximal portion 85 of the fluid pathways 316 is positioned between the two electrodes 302 and 308 and is herein illustrated by dashed lines to indicate that the test strip confines the fluid pathways therebetween. Accordingly, the electrodes of the test strip form barriers or walls for a portion of the fluid pathways such that sample flowing through the pathways is contacted by the electrodes while still in the pathway. As described above, an appropriate redox reagent system may be located in the fluid pathways to define another reaction area or zone of the test strip. In certain other embodiments, proximal portion 85 of the fluid pathways may not be open, i.e., in contact with the electrodes along their length.

Figure 4 shows a subject test strip, such as test strip 300 of Figure 3A, inserted into a meter 9, where the meter is capable of automatically determining the concentration of at least one analyte in a sample applied to the test strip.

### SYSTEMS

As mentioned above, the subject invention includes an analyte concentration determination system capable of obtaining a physiological sample and determining the analyte concentration of an analyte of interest therein, where determining the analyte concentration may be accomplished automatically by an automated device, e.g., a meter. Accordingly, the analyte concentration determination system of the subject invention includes a test strip having at least one subject skin-piercing element, as described above, associated therewith, and a meter (see Figure 4).

### METHODS

As summarized above, the subject invention provides methods for determining the concentration of an analyte in a sample. The subject methods find use in the determination of a variety of different analyte concentrations, where representative analytes include glucose, cholesterol, lactate, alcohol, and the like. In many embodiments, the subject methods are employed to determine the glucose concentration in a test fluid, e.g., a physiological sample.

While in principle the subject methods may be used to determine the concentration of an analyte in a variety of different physiological samples, such as urine, tears, saliva, and the like, they are particularly suited for use in determining the concentration of an analyte in blood or blood fractions, and more particularly in whole blood or interstitial fluid.

In practicing the subject methods, a skin-piercing element for accessing and collecting physiological sample with minimal pain is inserted into the skin and sample is collected through the at least one fluid pathway of the skin-piercing element, oftentimes by entering one or more sides of the fluid pathway, where such sides are open to the outside environment along at least a portion of their length, usually at least a substantial portion of the distal length of the fluid pathway and oftentimes the entire length of the fluid pathway on one or more sides or portions of the circumference. Following sample collection, the concentration of at least one analyte in the sample may then be determined. In other embodiments of the subject methods, sample is collected through one or more openings associated with the fluid pathway usually the distal end of the fluid pathway.

Thus, the first step in the subject methods is to provide at least one suitable skin-piercing element, such as one or more skin-piercing elements described above. In other words, the skin-piercing element has at least one fluid pathway therein, which is open on at least a substantial portion of its distal end to the outside environment, where it is open either along one or more of its sides or portion of the circumference (see for example Figures 1A-1F) or via at least one opening of the skin-piercing element (see for example Figures 2A-2C). In certain embodiments, a substantial portion of the distal end of the skin-piercing element is open to the outside environment. Usually, the at least one fluid pathway will terminate proximal to the distal tip of the skin-piercing element. The subject skin-piercing elements may be associated, affixed, integrated or attached to a test strip, as described above.

Depending on the type of physiological sample to be obtained, one or more of the subject skin-piercing elements may penetrate to various skin layers, including the dermis, epidermis and the stratum corneum, but in many embodiments will penetrate no farther than the subcutaneous layer of the skin.

Typically, the one or more skin-piercing element is inserted into the skin, generally into a finger or arm, e.g., a forearm, for about 1 to 60 seconds, usually about 1 to 15 seconds and more usually from about 1 to 5 seconds.

Once inserted into the skin, physiological sample is collected so that the concentration of an analyte of interest may be determined. Accordingly, once collected, the sample is then transferred to a test strip or the like, specifically to the reaction area of a test strip, where the reaction area may include the fluid pathway and/or other areas of the test strip, as described above.

More specifically, sample located at or near one or more entry points of the fluid pathway, e.g., one or more sides of the fluid pathway or one or more points or portion of the circumference of the fluid pathway which are open to the outside environment or the pathway openings or holes, is collected through such entry points. Thus, the skin is pierced by one or more of the skin-piercing elements, where the one or more skin-piercing elements penetrates to an appropriate layer of skin and draws physiological sample, e.g., sample located adjacent to the distal tips of the skin-piercing elements, into the one or more fluid pathways. In certain embodiments, the sample is drawn into the fluid pathway(s) by a capillary force exerted on the sample, typically exerted by the fluid pathway itself. The sample may then be transferred to one or more reaction areas, including reaction areas of the fluid pathway and/or test strip.

More specifically, in those embodiments where the at least one skin piercing element includes a fluid pathway that has at least a substantial portion of its distal end open to the outside environment on at least one side or portion of its circumference (see for example Figures 1A through 1F), the fluid pathway may exert a capillary force on physiological fluid located near the pathway opening and draw a volume of fluid into the entry points, i.e., the opened sides or portions of the fluid pathway. Thus, because at least a substantial portion of the distal end of the fluid pathway is exposed to the outside environment, it can be appreciated that a greater volume of fluid per unit time can be collected from a greater area when compared to a conventional needle which typically has a single opening at its distal tip.

In those embodiments where the at least one skin-piercing element includes a fluid pathway in association with at least one side opening, the fluid pathway may exert a capillary force on physiological fluid located near the one or more openings and draw a volume of fluid into the openings of the fluid pathway. Again, this configuration enables a greater volume of fluid per unit time to be collected from a greater area when compared to a conventional needle which typically has a single opening at its distal tip.

As described above, once sample is collected and contacted with the reaction area of a test strip, the concentration of the analyte of interest is determined. In certain methods, analyte concentration determination may occur or commence in the fluid pathway. For example, where a portion of the fluid pathway is formed by the test strip, e.g., one or more electrodes, and includes a redox reagent system located at least in the fluid pathway, the concentration determination of an analyte can occur while sample is still in the pathway, i.e., the reaction need not wait to commence at a remote reaction site, instead of, or in addition to, commencing or occurring in other reaction areas of the test strip. Of course, sample may be transferred to a reaction area of the test strip other than a reaction area within a fluid pathway, i.e., a remote reaction area. For example, sample may be transferred or delivered to a reaction area located at the proximal end of a fluid pathway.

Regardless of where the reaction area(s) is positioned, as mentioned above for an electrochemical analyte concentration determination assay, an electrochemical measurement is made using the reference and working electrodes. The electrochemical measurement that is made may vary depending on the particular nature of the assay and the test strip with which the electrochemical test strip is employed, e.g., depending on whether the assay is coulometric, amperometric or potentiometric. Generally, the electrochemical measurement will measure charge (coulometric), current (amperometric) or potential (potentiometric), usually over a given period of time following sample introduction into the reaction area. Methods for making the above described electrochemical measurement are further described in U.S. Patent Nos.: 4,224,125; 4,545,382; and 5,266,179; as well as WO 97/18465; WO 99/49307; the disclosures of the priority documents of which are herein incorporated by reference. Regardless of the type of measurement, an electrochemical measurement or signal is made in the reaction zone of the test strip, where, as noted above, the reaction zone may include the fluid pathway and/or alternative test strip areas. In many embodiments, the measurement may occur first or begin in the fluid pathway and then also in an alternative site.

Following detection of the electrochemical measurement or signal generated in the reaction zone as described above, the presence and/or concentration of the analyte present in the sample introduced into the reaction zone is then determined by relating the electrochemical signal to the amount of analyte in the sample.

As described above, the subject test strip may be configured and adapted to be inserted into a meter and, in many embodiments, the above described determination and relation processes are performed by an automated meter, as is well known in the relevant art (see Figure 4 which illustrates a subject test strip 10 inserted into a meter 9). Representative meters for automatically practicing these steps are further described in
EP-A-1 067 384, WO 01/57510, WO 01/57238. Typically, the meter display the analyte concentration to the user via a display window or panel of the meter.

For a colorimetric or photometric analyte concentration determination assay, sample applied to the test strip, more specifically to a reaction area of a test strip, is allowed to react with members of a signal producing system to produce a detectable product that is present in an amount proportional to the initial amount present in the sample. The amount of detectable product, i.e., signal produced by the signal producing system, is then determined and related to the amount of analyte in the initial sample. As described, in certain embodiments, automated meters, i.e., optical meters, that perform the above mentioned detection and relation steps are employed. The above described reaction, detection and relating steps, as well as instruments for performing the same, are further described in U.S. Patent Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference. Examples of such colorimetric or photometric reagent test strips suitable for use with the subject invention include those described in U.S. Patent Nos.: 5,563,042; 5,753,452; 5,789,255, herein incorporated by reference.

### KITS

Also provided by the subject invention are kits for use in practicing the subject methods. The kits of the subject invention include at least one subject skin-piercing element, oftentimes a plurality of skin-piercing elements, where the at least one skin-piercing element may be associated with a test strip. The kits may include a plurality of such skin-piercing elements and/or such test strips. The kits may also include a reusable or disposable meter that may be used with reusable or disposable tests trips of the kit or from other kits or the subject invention. Certain kits may include various types of test strips, e.g., where various test strips contain the same or different reagents, e.g., electrochemical and/or colorimetric test strips, or the same or different skin-piercing elements, e.g., single or multiple fluid pathways, etc. Finally, the kits may further include instructions for using the subject test strips in the determination of an analyte concentration in a physiological sample. These instructions may be present on one or more of the packaging, a label insert, containers in the kits, and the like.

It is evident from the above description and discussion that the above described invention provides a simple, quick and convenient way to obtain a physiological sample and determine an analyte concentration thereof. The above described invention provides a number of advantages, including ease of use, decreased testing times, efficiency and minimal pain. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## Claims

1. A skin-piercing element for piercing the skin, said skin-piercing element comprising at least one fluid pathway, wherein at least a substantial portion of the distal end of said at least one fluid pathway is open to the outside environment.

2. The skin-piercing element according to claim 1, wherein said substantial portion of the distal end of said at least one fluid pathway is open to the outside environment on one or more sides or along any point of the circumference of said at least one fluid pathway.

3. The skin-piercing element according to claim 1, wherein the entire length of said distal end of said at least one fluid pathway is open to the outside environment on said one or more sides or along any point of the circumference of said skin-piercing element.

4. The skin-piercing element according to claim 1, wherein said at least one fluid pathway terminates proximal to the distal tip.

5. The skin-piercing element according to claim 1, comprising a plurality of fluid pathways.

6. A test strip for determining at least one target analyte concentration of a physiological sample, said test strip comprising at least one skin-piercing element according to claim 1.

7. The test strip according to claim 6, wherein a portion of said fluid pathway is formed by said test strip.

8. The test strip according to claim 6, wherein said test strip further comprises two electrodes and said portion is formed by at least one of said electrodes of said test strip.

9. The test strip according to claim 8, wherein said test strip further comprises a spacer layer between said electrodes and said portion is formed by said spacer layer.

10. A system for determining at least one target analyte concentration of a physiological sample, said system comprising:
a test strip according to claim 6, and
a meter for automatically determining the concentration of analyte in said physiological sample.

11. A method for collecting physiological sample, said method comprising:
(a) providing at least one skin-piercing element comprising at least one fluid pathway, wherein at least a substantial portion of the distal end of said at least one fluid pathway is open to the outside environment;
(b) inserting said at least one skin-piercing element into the skin; and
(c) collecting by means of said at least one fluid pathway said physiological sample from within the skin.
